# EUROPÄISCHE PATENTANMELDUNG

(11) **EP 3 327 437 A2**
(43) Veröffentlichungstag der Anmeldung: **30.05.2018**
(21) Anmeldenummer: 17198233.3
(22) Anmeldetag: 25.10.2017
(51) Int. Cl.: G01N 33/28, G01N 15/14

(54) **VERFAHREN UND VORRICHTUNG ZUM ERMITTELN VON FESTSTOFFPARTIKELN IN EINEM FLÜSSIGKEITSSTROM**

(30) Priorität: 23.11.2016 DE 102016223221
(71) Anmelder: Filtration Group GmbH, 74613 Öhringen (DE)
(72) Erfinder: CHRISTMANN, Lars, 74081 Heilbronn (DE); RÜCK, Ernst, 74629 Pfedelbach (DE); WIERLING, Reinhard, 74629 Pfedelbach (DE)
(74) Vertreter: BRP Renaud & Partner mbB Rechtsanwälte Patentanwälte Steuerberater

(57) **Zusammenfassung**

Die vorliegende Erfindung betrifft ein Verfahren zum Ermitteln eines Gehalts an Feststoffpartikeln in einem Flüssigkeitsstrom, vorzugsweise in einem Hydrauliksystem mit folgenden Schritten:
- Reduzieren eines Gasgehalts an Gaspartikeln im Flüssigkeitsstrom,
- Komprimieren der Gaspartikel im Flüssigkeitsstrom.

## Beschreibung

Die vorliegende Erfindung betrifft ein Verfahren sowie eine Vorrichtung zum Ermitteln eines Gehalts an störenden Feststoffpartikeln in einem Flüssigkeitsstrom, vorzugsweise in einem Hydrauliksystem. Die Erfindung betrifft außerdem eine Maschine, die mit einer solchen Vorrichtung ausgestattet ist.

Eine Vielzahl von Maschinen arbeitet mit einem oder mehreren Hydrauliksystemen, insbesondere Hydraulikkreisen. Von besonderer Bedeutung sind dabei Schmierölkreise. Hierbei kann es sich grundsätzlich um mobile Maschinen, wie z. Bsp. Antriebsaggregate, insbesondere Getriebe, Kurbelwelle, Nockenwellen, in Fahrzeugen, sowie stationäre Maschinen handeln, wie z. Bsp. Getriebe von Windkraftanlagen oder Förderanlagen. Der Gehalt an Feststoffpartikeln im Flüssigkeitsstrom eines solchen Hydrauliksystems korreliert mit einem Verschleiß bzw. mit einem Betriebszustand der zugehörigen Maschine. Dementsprechend ist es für die Funktionsüberwachung bzw. für die Wartung einer derartigen Maschine von entscheidender Bedeutung, den Gehalt an Feststoffpartikeln möglichst genau bestimmen zu können. In einem derartigen Flüssigkeitsstrom sind jedoch regelmäßig auch Gaspartikel enthalten, die von herkömmlichen Sensoren nicht von Feststoffpartikeln unterschieden werden können, so dass bei herkömmlicher Vorgehensweise häufig ein zu hoher Gehalt an Partikeln im Flüssigkeitsstrom gemessen wird.

Aus der DE 10 2012 016 458 A1 ist eine Vorrichtung zum Ermitteln eines Gehalts an störenden Feststoffpartikeln in einem Flüssigkeitsstrom bekannt. Die bekannte Vorrichtung umfasst eine Mess-Strecke, die einen Partikelsensor zum Feststellen des Partikeleintrags aufweist, und kennzeichnet sich durch eine Probenaufbereitungs-Strecke aus, die mittels einer Anschlusseinrichtung fluidführend an die Mess-Strecke anschließbar ist. Diese Probenaufbereitungs-Strecke weist zumindest eine zusätzliche Einrichtung auf, mit deren Hilfe weitere fluidfremde Verunreinigungen, insbesondere Gaspartikel, festgestellt und/oder zumindest teilweise aus dem Flüssigkeitsstrom ausgeschieden werden können. Die bekannte Vorrichtung nutzt somit einen zusätzlichen Sensor zur Bestimmung der Gaspartikel. Dies kann mit erhöhten Kosten verbunden sein.

Aus der DE 10 2009 048 271 A1 ist eine weitere Vorrichtung zum Ermitteln eines Gehalts an störenden Feststoffpartikeln in einem Flüssigkeitsstrom bekannt, bei dem eine Pumpe verwendet wird, die über einen internen Leckageanschluss gashaltige Flüssigkeit einem Flüssigkeitstank rückführt. In der Pumpe erfolgt ein Komprimiervorgang, der innerhalb der Pumpe die Gasblasen ebenfalls dem internen Leckageanschluss zuführt. Ferner werden die komprimierten Gaspartikel im Flüssigkeitsstrom gelöst, so dass das Gas nicht mehr in Partikelform vorliegt. Ferner wird eine Fluidleitung zum Führen des Flüssigkeitsstroms hinsichtlich ihrer Dimensionierung so gewählt, dass sich darin ein Beruhigungseffekt einstellt, der die Lösung der komprimierten Gaspartikel im Flüssigkeitsstrom unterstützt. Auf diese Weise wird erreicht, dass letztlich am Sensor nur noch Feststoffpartikel im Flüssigkeitsstrom enthalten sind. Dementsprechend zählt der Sensor nur noch die Feststoffpartikel. Diese Vorrichtung geht somit davon aus, dass stromauf des Sensors zum Bestimmen der Feststoffpartikel sämtliche Gaspartikel aus dem Flüssigkeitsstrom entfernt werden können. Hierzu ist ein vergleichsweise großer apparativer Aufwand erforderlich. Außerdem lässt sich nicht ausschließen, dass doch noch ein gewisser Anteil an Gaspartikeln im Flüssigkeitsstrom verbleibt und dementsprechend vom Sensor mitgezählt wird.

Aus der DE 10 2012 013 255 A1 ist ein Verfahren bekannt, bei dem Feststoffpartikel und Gaspartikel anhand unterschiedlicher charakteristischer Messwertabfolgen voneinander unterschieden werden können. Bei diesem Verfahren wird ein hoch komplexer Sensor verwendet, wodurch auch hier der Aufwand zur Realisierung vergleichsweise groß ist.

Die vorliegende Erfindung beschäftigt sich mit dem Problem, für ein Verfahren bzw. für eine Vorrichtung der vorstehend beschriebenen Art eine verbesserte Ausführungsform anzugeben, die sich insbesondere durch eine preiswerte Realisierbarkeit auszeichnet.

Dieses Problem wird durch die Gegenstände der unabhängigen Ansprüche gelöst. Vorteilhafte Ausführungsformen sind Gegenstand der abhängigen Ansprüche.

Gemäß einem ersten Aspekt beruht die vorliegende Erfindung auf dem allgemeinen Gedanken, den Partikelgehalt an Feststoffpartikeln und Gaspartikeln im Flüssigkeitsstrom zu messen und diese Messung anschließend hinsichtlich ihrer Plausibilität zu überprüfen. Für diese Plausibilitätsprüfung schlägt dieser erste Aspekt der Erfindung vor, zusätzlich den Gasgehalt im Flüssigkeitsstrom zu ermitteln. Durch die Kenntnis des Gasgehalts im Flüssigkeitsstrom lässt sich die Zuverlässigkeit des ermittelten Partikelgehalts im Flüssigkeitsstrom bewerten. Je geringer der Gasgehalt im Flüssigkeitsstrom, desto zuverlässiger ist der ermittelte Partikelgehalt im Flüssigkeitsstrom. Um mit einer erhöhten Wahrscheinlichkeit zuverlässige bzw. plausible Messergebnisse zu erhalten, wird außerdem vorgeschlagen, vor der Messung im Flüssigkeitsstrom den Gehalt an Gaspartikeln zu reduzieren, z.B. durch Entgasung und/oder durch Lösung des Gases in der Flüssigkeit. Zusätzlich kann optional vorgesehen sein, den Flüssigkeitsstrom zu komprimieren, um einerseits weiter das Lösen der Gase in der Flüssigkeit zu unterstützen, und andererseits die Größe der Gaspartikel zu reduzieren. Diese Maßnahme beruht auf der Erkenntnis, dass sich bei gleicher Anzahl an Gaspartikeln kleinere Gaspartikel weniger nachteilig auf die Messung des Partikelgehalts auswirken als größere Gaspartikel.

Gemäß einer vorteilhaften Ausführungsform wird ein Gasgehaltgrenzwert festgelegt bzw. vorbestimmt, der zur Überprüfung der Plausibilität herangezogen werden kann. Ist der ermittelte Gasgehalt unterhalb dieses Gasgehaltgrenzwerts, besitzt der ermittelte Partikelgehalt eine ausreichende Zuverlässigkeit. Übersteigt dagegen der ermittelte Gasgehalt den vorgegebenen Gasgehaltgrenzwert ist der ermittelte Partikelgehalt zu unzuverlässig, um einen zutreffenden Rückschluss über den Zustand der Flüssigkeit bzw. des Hydrauliksystems bzw. der zugehörigen Maschine geben zu können.

Vorzugsweise lässt sich mit Hilfe eines optischen Sensors, der eine Streulichtmessung durchführt, der Gasgehalt im Flüssigkeitsstrom ermitteln. Dies beruht auf der Erkenntnis, dass Gaspartikel eine andere Streulichtcharakteristik besitzen als Feststoffpartikel. Der Partikelgehalt insgesamt, also unabhängig davon, ob es sich um Gaspartikel oder Feststoffpartikel handelt, lässt sich in konventioneller Weise ermitteln. Beispielsweise kann auch hier ein optischer Sensor zum Einsatz kommen, der den Anteil an absorbiertem Licht ermittelt bzw. ein Exstinktionsverfahren durchführt.

Gemäß einem zweiten Aspekt, der alternativ oder kumulativ zum vorstehend erläuterten ersten Aspekt verwirklicht werden kann, beruht die Erfindung auf dem allgemeinen Gedanken, zusätzlich zur Anzahl der Partikel außerdem die Größe der Partikel zu messen. Ferner ist wesentlich, dass die Bestimmung der Partikelgröße in einem komprimierten Zustand erfolgt. Die Erfindung nutzt hierbei die Erkenntnis, dass der Flüssigkeitsstrom und die Feststoffpartikel inkompressibel sind, während die Gaspartikel kompressibel sind. In der Folge werden durch die Kompression nur die Gaspartikel in ihrer Größe reduziert. Ferner wurde gemäß diesem zweiten Aspekt erkannt, dass nicht sämtliche Feststoffpartikel kritisch für die jeweilige Maschine sind, sondern erst ab einer vorbestimmten Partikelgrößengrenze. Mit anderen Worten, es reicht aus, den Gehalt an störenden Feststoffpartikeln im Flüssigkeitsstrom zu ermitteln, wobei sich die störenden Feststoffpartikel dadurch charakterisieren, dass ihre Partikelgröße größer ist als die vorgenannte Partikelgrößengrenze. Durch eine gezielte Abstimmung der vorstehend genannten Kompression der Gaspartikel wird erreicht, dass die komprimierten Gaspartikel eine Partikelgröße erreichen, die unterhalb der Partikelgrößengrenze liegt. Mit anderen Worten, die komprimierten Gaspartikel werden nicht als störende Partikel identifiziert, unabhängig davon, ob sie fest oder gasförmig sind. Zusammengefasst werden gemäß diesem zweiten Aspekt beim erfindungsgemäßen Verfahren bzw. in der zugehörigen Vorrichtung nur der Gehalt an störenden Partikeln ermittelt, wobei durch Kompression der Gaspartikel erreicht wird, dass im Flüssigkeitsstrom enthaltene Gaspartikel kleiner sind als eine vorbestimmte Partikelgrößengrenze. Nur Partikel deren Partikelgröße oberhalb dieser Partikelgrößengrenze liegt, werden als störend identifiziert. Alle anderen Partikel, unabhängig davon, ob es sich um Feststoffpartikel oder Gaspartikel handelt, werden als nicht störend eingestuft.

Bei beiden vorstehend beschriebenen Aspekten kommen das erfindungsgemäße Verfahren und die zugehörige Vorrichtung somit ohne eine hochkomplexe Sensorik aus. Gleichzeitig kann mit relativ hoher Zuverlässigkeit der Gehalt an störenden Feststoffpartikeln ermittelt werden. Ebenso ist der erforderliche apparative Aufwand vergleichsweise gering.

Vorzugsweise kann vor dem Komprimieren der Gaspartikel im Flüssigkeitsstrom außerdem ein Reduzieren des Gehalts an Gaspartikeln im Flüssigkeitsstrom durchgeführt werden. Durch ein derartiges Reduzieren des Gehalts an Gaspartikeln im Flüssigkeitsstrom werden vor allem Gaspartikel mit größerer Partikelgröße aus dem Flüssigkeitsstrom entfernt. Dies kann durch ein Entgasen erfolgen. Ebenso kann dies durch ein Auflösen der Gaspartikel im Flüssigkeitsstrom erfolgen. Durch diesen Auflösungsvorgang wird zumindest die Größe der Gaspartikel reduziert.

Vorzugsweise kommt zum Reduzieren des Gehalts an Gaspartikeln im Flüssigkeitsstrom daher bspw. eine Entgasungseinrichtung zum Einsatz. Diese kann optional mit einer Beruhigungsstrecke kombiniert sein, um durch Lösen des Gases in der Flüssigkeit die Partikelgröße der Gaspartikel weiter zu reduzieren.

Vorzugsweise erfolgt das Komprimieren der Gaspartikel im Flüssigkeitsstrom stromab der Entgasungseinrichtung. Dies vereinfacht den Aufbau der Entgasungseinrichtung.

Bei einer anderen Ausführungsform kann das Komprimieren der Gaspartikel im Flüssigkeitsstrom durch eine Druckerhöhung im Flüssigkeitsstrom erfolgen. Beispielsweise lässt sich eine derartige Druckerhöhung durch eine Pumpe oder dergleichen realisieren. Vorzugsweise ist diese Pumpe im Flüssigkeitsstrom stromab der Entgasungseinrichtung angeordnet.

Die Bestimmung des Gehalts der störenden Feststoffpartikel im Flüssigkeitsstrom kann gemäß einer bevorzugten Ausführungsform dadurch erfolgen, dass nur die Anzahl der als störend identifizierten Feststoffpartikeln gezählt wird. Die gemessenen Partikel werden dann als störende Feststoffpartikel identifiziert, wenn die zugehörige Partikelgröße größer ist als die vorbestimmte Partikelgrößengrenze. Ist dagegen die zugehörige Partikelgröße kleiner als die Partikelgrößengrenze, wird das jeweilige Partikel als nicht störend bzw. als Gaspartikel identifiziert. Wenn nur die als störend identifizierten Feststoffpartikel gezählt werden, ist der erforderliche apparative Aufwand vergleichsweise gering.

Alternativ kann der Gehalt störender Feststoffpartikel im Flüssigkeitsstrom auch dadurch bestimmt werden, dass zunächst die Gesamtzahl aller Feststoffpartikel und Gaspartikel im Flüssigkeitsstrom gezählt wird und dass anschließend von dieser Gesamtzahl die Anzahl an Partikeln subtrahiert wird, die als nicht störend identifiziert werden. Mit anderen Worten, es werden alle Gaspartikel und Feststoffpartikel abgezogen, deren Partikelgrößer kleiner als die Partikelgrößengrenze ist. Bei dieser Vorgehensweise können die Zählung der Partikel und die Größenbestimmung der Partikel grundsätzlich auseinander fallen, was zu einer Vereinfachung im Aufbau einer zugehörigen Vorrichtung führen kann.

Vorzugsweise kann die Anzahl der Partikel mittels eines optischen Sensors ermittelt werden. Auch die Partikelgröße kann zweckmäßig mittels eines optischen Sensors ermittelt werden. Besonders vorteilhaft können die Partikelgröße und die Partikelanzahl mit demselben Sensor ermittelt werden, der vorzugsweise optisch arbeitet. Ebenso ist denkbar, einen Sensor vorzusehen, der die Partikel unabhängig von ihrer Größe zählt, und einen weiteren Sensor vorzusehen, der entweder nur die Partikel zählt, deren Partikelgröße oberhalb der Partikelgrößengrenze liegt, oder nur diejenigen Partikel zählt, deren Partikelgröße unterhalb der Partikelgrößengrenze liegt.

Eine erfindungsgemäße Vorrichtung umfasst eine Reduzierungseinrichtung zum Reduzieren eines Gasgehalts an Gaspartikeln im Flüssigkeitsstrom, eine stromab der Reduzierungseinrichtung angeordnete Komprimierungseinrichtung zum Komprimieren der Gaspartikel im Flüssigkeitsstrom, eine stromab der Komprimierungseinrichtung angeordnete Messeinrichtung und eine Auswerteeinrichtung, die mit der Messeinrichtung gekoppelt ist und die so ausgestaltet bzw. programmiert ist, dass sie im Betrieb der Vorrichtung das vorstehend beschriebene Verfahren ausführen kann.

Eine erfindungsgemäße Maschine weist zumindest ein Hydrauliksystem auf und ist mit wenigstens einer Vorrichtung der vorstehend beschriebenen Art ausgestattet.

Weitere wichtige Merkmale und Vorteile der Erfindung ergeben sich aus den Unteransprüchen, aus den Zeichnungen und aus der zugehörigen Figurenbeschreibung anhand der Zeichnungen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Bevorzugte Ausführungsbeispiele der Erfindung sind in den Zeichnungen dargestellt und werden in der nachfolgenden Beschreibung näher erläutert, wobei sich gleiche Bezugszeichen auf gleiche oder ähnliche oder funktional gleiche Bauteile beziehen.

Es zeigen, jeweils schematisch,
- Fig. 1 und 2: jeweils eine stark vereinfachte, schaltplanartige Prinzipdarstellung einer Maschine, die mit einer erfindungsgemäßen Vorrichtung ausgestattet ist, bei verschiedenen Ausführungsformen,
- Fig. 3 und 4: jeweils eine stark vereinfachte, schaltplanartige Prinzipdarstellung der erfindungsgemäßen Vorrichtung bei verschiedenen Ausführungsformen.

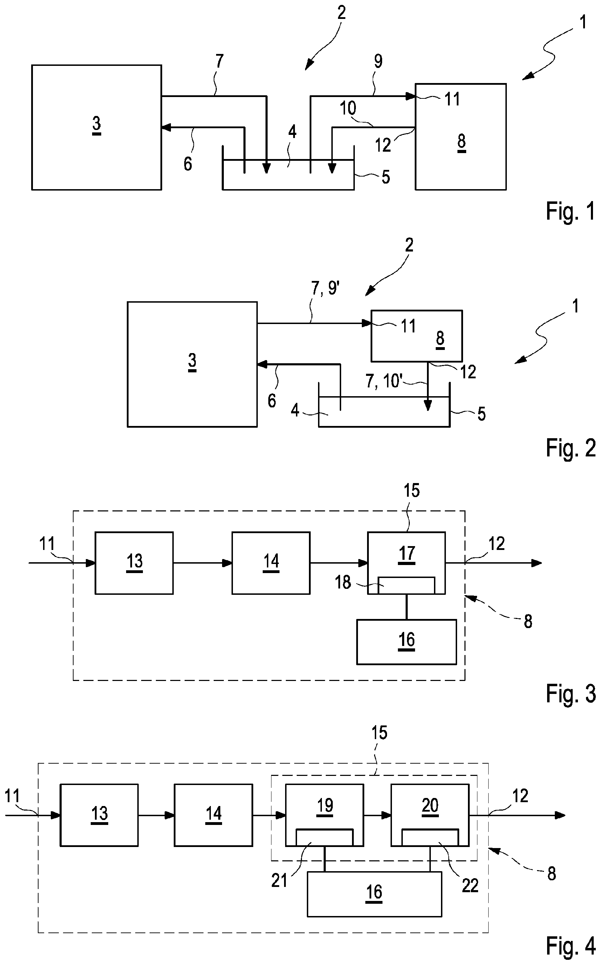

Entsprechend den Fig. 1 und 2 umfasst eine Maschine 1 ein Hydrauliksystem 2 zur Versorgung einer Komponente 3 der Maschine 1 mit einer Flüssigkeit 4. Beispielsweise handelt es sich bei der Maschine 1 um ein Kraftfahrzeug oder um eine Brennkraftmaschine. Die Komponente 3 kann bspw. ein Getriebe sein. Die Flüssigkeit 4 ist dann zweckmäßig ein Getriebeöl. Ebenso kann ein Kurbeltrieb der Brennkraftmaschine eine solche Komponente 3 bilden, die mit Schmieröl versorgt wird, oder ein Ventiltrieb mit mindestens einer Nockenwelle, der mit Schmieröl versorgt wird.

Das Hydrauliksystem 2 umfasst einen Tank 5 bzw. ein Reservoir 5 für die Flüssigkeit 4. Vom Reservoir 5 führt eine Vorlaufleitung 6 zur jeweiligen Komponente 3. Eine Rücklaufleitung 7 führt von der jeweiligen Komponente 3 zum Reservoir 5 zurück.

Die Maschine 1 ist außerdem mit einer Vorrichtung 8 zum Ermitteln eines Gehalts an störenden Feststoffpartikeln in einem Flüssigkeitsstrom ausgestattet. Die Vorrichtung 8 misst dabei den Gehalt an Feststoffpartikeln in einem Flüssigkeitsstrom aus der Flüssigkeit 4 und liefert einer hier nicht gezeigten Steuerung der Maschine 1 dann einen Wert, der mit dem aktuellen Gehalt an Feststoffpartikeln in der Flüssigkeit korreliert. Üblicherweise wird dieser Gehalt in der Flüssigkeit in PPM pro Volumeneinheit angegeben, bspw. PPMV, PPBV oder PPTV. Ebenso ist eine Angabe in VPM möglich. Grundsätzlich ist anstelle eines Volumenbezugs auch ein Massebezug denkbar, also bspw. PPMW, PPBW oder PPTW.

Gemäß Fig. 1 kann dieser Vorrichtung 8 der Flüssigkeitsstrom über eine Zuführleitung 9 vom Reservoir 5 zugeführt werden. Über eine Rückführleitung 10 kann dann die Rückführung des Flüssigkeitsstroms von der Vorrichtung 8 zum Reservoir 5 erfolgen. Fig. 1 zeigt somit eine Parallelschaltung der Komponente 3 einerseits und der Vorrichtung 8 andererseits im Hydrauliksystem 2. Im Unterschied dazu zeigt Fig. 2 eine Reihenschaltung von Komponente 3 einerseits und Vorrichtung 8 andererseits im Hydrauliksystem 2. Hierzu ist die Vorrichtung 8 rein exemplarisch in die Rücklaufleitung 7 eingebunden. Hierdurch wird die Rücklaufleitung 7 in einen von der Komponente 3 zur Vorrichtung 8 führenden Zuführabschnitt 9' und einen von der Vorrichtung 8 zum Reservoir 5 führenden Rückführabschnitt 10' unterteilt. In der Darstellung der Fig. 2 wird der gesamte Flüssigkeitsstrom, der die Komponente 3 durchströmt, auch durch die Vorrichtung 8 geführt. Es ist klar, dass bei einer anderen Ausführungsform vorgesehen sein kann, dass nur ein Teil des Flüssigkeitsstroms, der durch die Komponente 3 hindurch geführt wird, für die Messung des Gehalts an Feststoffpartikeln durch die Vorrichtung 8 geführt wird. In diesem Fall ist die Vorrichtung 8 dann in einem Zweig der Rücklaufleitung 7 angeordnet.

Jedenfalls weist die Vorrichtung 8 einen Einlass 11 für den Flüssigkeitsstrom und einen Auslass 12 für den Flüssigkeitsstrom auf. Im Beispiel der Fig. 1 ist an den Einlass 11 die Zuführleitung 9 angeschlossen, während an den Auslass 12 die Rückführleitung 10 angeschlossen ist. Im Beispiel der Fig. 2 ist an den Einlass 11 der Zuführabschnitt 9' der Rücklaufleitung 7 angeschlossen. An den Auslass 12 ist der Rückführabschnitt 10' der Rücklaufleitung 7 angeschlossen.

Entsprechend den Fig. 3 und 4 umfasst die Vorrichtung 8 eine Reduzierungseinrichtung 13 zum Reduzieren eines Gehalts an Gaspartikeln im Flüssigkeitsstrom. Ferner umfasst die Vorrichtung 8 eine Komprimierungseinrichtung 14 zum Komprimieren der Gaspartikel im Flüssigkeitsstrom. Dabei ist die Komprimierungseinrichtung 14 stromab der Reduzierungseinrichtung 13 angeordnet.

Desweiteren ist die Vorrichtung 8 mit einer Messeinrichtung 15 ausgestattet, mit deren Hilfe im Allgemeinen der Gehalt an Feststoffpartikeln im Flüssigkeitsstrom gemessen werden kann. Im Einzelnen kann die Messeinrichtung 15 gemäß einem ersten Aspekt der Erfindung den Partikelgehalt ermitteln und eine Plausibilitätsprüfung für den gemessenen Wert durchführen. Zusätzlich oder alternativ kann die Messeinrichtung 15 gemäß einem zweiten Aspekt der Erfindung die Partikelanzahl und die Partikelgröße der Feststoffpartikel und der komprimierten Gaspartikel im Flüssigkeitsstrom messen. Ferner ist eine Auswerteeinrichtung 16 vorgesehen, die mit der Messeinrichtung 15 gekoppelt ist. Diese Auswerteeinrichtung 16 ist so konfiguriert, dass sie das weiter unten näher erläuterte Verfahren ausführen kann.

Die Messeinrichtung 15 enthält im Beispiel der Fig. 3 in einer einzigen Messstation 17 einen optischen Sensor 18, der gemäß dem ersten Aspekt sowohl den Partikelgehalt im Flüssigkeitsstrom als auch den Gasgehalt im Flüssigkeitsstrom ermitteln kann. Gemäß dem zweiten Aspekt kann dieser Sensor 18 sowohl die Partikelanzahl als auch die Partikelgröße im Flüssigkeitsstrom ermitteln. Im Unterschied dazu zeigt Fig. 4 eine Ausführungsform, bei der die Messeinrichtung 15 gemäß dem ersten Aspekt zwei separate Messstationen 19, 20 aufweist, in denen jeweils ein optischer Sensor 21 bzw. 22 angeordnet ist. Einer der Sensoren 21, 22 kann zum Messen des Partikelgehalts im Flüssigkeitsstrom ausgelegt sein. Der andere Sensor 21, 22 kann zum Messen des Gasanteils im Flüssigkeitsstrom ausgelegt sein. Anstelle von zwei separaten Messstellen 19, 20 kann auch hier eine gemeinsame Messstelle wie im Beispiel der Fig. 3 vorgesehen sein, nur dass darin ebenfalls zwei separate Sensoren 21, 22 zum Einsatz kommen.

Nachfolgend wird das Verfahren zum Ermitteln des Gehalts an störenden Feststoffpartikeln im Flüssigkeitsstrom näher erläutert, das mit Hilfe der vorstehend beschriebenen Vorrichtung 8 ausführbar ist.

Zunächst wird der Gehalt an Gaspartikeln im Flüssigkeitsstrom reduziert. Dies erfolgt zweckmäßig in der Reduzierungseinrichtung 13. Hierzu kann die Reduzierungseinrichtung 13 eine Entgasungseinrichtung und/oder eine Beruhigungsstrecke enthalten. Während die Entgasungseinrichtung Gaspartikel aus dem Flüssigkeitsstrom ausscheidet, erfolgt in der Beruhigungsstrecke ein Lösen des Gases der Gaspartikel in der Flüssigkeit des Flüssigkeitsstroms. Somit reduziert sich die Partikelgröße der Gaspartikel. Anschließend erfolgt ein Komprimieren der Gaspartikel im Flüssigkeitsstrom. Dies erfolgt in der Komprimierungseinrichtung 14. Beispielsweise kann die Komprimierungseinrichtung 14 hierzu eine Pumpe aufweisen oder durch eine Pumpe gebildet sein.

Gemäß dem ersten Aspekt des hier vorgestellten Verfahrens erfolgt anschließend in der Messeinrichtung 15 ein Ermitteln des Partikelgehalts im Flüssigkeitsstrom. Dabei werden sowohl Feststoffpartikeln als auch Gaspartikeln im Flüssigkeitsstrom erfasst. Dementsprechend kann hier ein einfacher, konventioneller Sensor zum Einsatz kommen, der beispielsweise als Trübungssensor ausgestaltet ist und nur das durchgehende Licht erfasst und mit dem abgestrahlten Licht vergleicht. Es ist klar, dass dieser Vergleich von abgestrahltem Licht und ankommendem Licht zur Ermittlung des Partikelgehalts letztlich auch in der Auswerteeinrichtung 16 erfolgen kann. Außerdem erfolgt, insbesondere in der Messeinrichtung 15, ein Ermitteln des Gasgehalts im Flüssigkeitsstrom. Mit Hilfe eines optischen Sensors 18 bzw. 21 bzw. 22 kann bspw. eine Streulichtmessung durchgeführt werden, um den Gasgehalt in der Flüssigkeitsströmung zu bestimmen. Die Streulichtcharakteristik von Gaspartikeln im Flüssigkeitsstrom unterscheidet sich signifikant von der Streulichtcharakteristik von Feststoffpartikeln im Flüssigkeitsstrom. Somit lässt sich durch Ermitteln des für die Gaspartikel charakteristischen Streulichts der Gasgehalt in der Flüssigkeitsströmung ermitteln. Ferner lässt sich mit dem jeweiligen optischen Sensor 18 bzw. 21 bzw. 22, insbesondere mittels Exstinktionsverfahren bzw. mittels Lichtabsorption der Partikelgehalt in der Flüssigkeitsströmung ermitteln, wobei die Messung mittels Exstinktion bzw. Absorption unabhängig davon ist, ob die Partikel fest oder gasförmig sind. Somit werden mit dem Exstinktionsverfahren bzw. Absorptionsverfahren Feststoffpartikel und Gaspartikel gemeinsam erfasst, so dass der gesamte Partikelgehalt an festen und gasförmigen Partikeln in der Flüssigkeitsströmung ermittelt wird. Auch hier ist klar, dass grundsätzlich die Messungen in der Messeinrichtung 15 erfolgen, während die sich daraus ergebenden Ermittlungen für Partikelgehalt und/oder Gasgehalt in der Auswerteeinrichtung 16 erfolgen können.

Anschließend wird vorzugsweise mit Hilfe der Auswerteeinrichtung 16 eine Plausibilitätsprüfung durchgeführt. Hierzu wird die Plausibilität des ermittelten Partikelgehalts anhand des Gasgehalts abgeschätzt bzw. bewertet. Wenn der ermittelte Gasgehalt unterhalb eines vorbestimmten Gasgehaltgrenzwerts liegt, wird der ermittelte Partikelgehalt als plausibel aufgefasst. Übersteigt dagegen der ermittelte Gasgehalt den vorbestimmten Gasgehaltgrenzwert, wird der ermittelte Partikelgehalt als nicht plausibel bzw. unzuverlässig bewertet. In der Folge können Maßnahmen zur Reduzierung des Gasgehalts angestoßen werden. Ebenso kann eine neue Messung angestoßen werden.

Gemäß dem zweiten Aspekt des Verfahrens kann eine Identifikation der Feststoffpartikel und der Gaspartikel anhand ihrer Partikelgröße durchgeführt werden. Genauer gesagt werden anhand der Partikelgröße störende Feststoffpartikel von nicht störenden Feststoffpartikeln und per se nicht störenden Gaspartikeln unterschieden. Störende Feststoffpartikel liegen vor, wenn die Partikel eine Partikelgröße aufweisen, die größer als eine vorbestimmte Partikelgrößengrenze ist. Ist die Partikelgröße dagegen kleiner als die vorbestimmte Partikelgrößengrenze, handelt es sich entweder um nicht störende Feststoffpartikel oder um Gaspartikel, die ebenfalls nicht stören. Unter Berücksichtigung der so ermittelten störenden Feststoffpartikel lässt sich nun der Gehalt der störenden Feststoffpartikel im Flüssigkeitsstrom bestimmen. Dies kann entweder dadurch erfolgen, dass bei der Ermittlung des Gehalts an Feststoffpartikeln im Flüssigkeitsstrom nur die störenden Feststoffpartikel berücksichtigt werden, also diejenigen Partikel, deren Partikelgröße oberhalb der Partikelgrößengrenze liegt. Ebenso ist denkbar, mit einem konventionellen Sensor zunächst den Gesamtgehalt der Partikel zu ermitteln, um dann anhand der Größenüberwachung den Anteil an störenden Partikeln bzw. den Anteil an nicht störenden Partikeln herauszufiltern. Beispielsweise lässt sich feststellen, in welchem Verhältnis die störenden Partikeln zu den nicht störenden Partikeln in der Flüssigkeitsströmung auftreten. In diesem Verhältnis kann dann die Messung des Gesamtgehalts der Partikel in der Flüssigkeitsströmung gewichtet werden.

## Patentansprüche

1. Verfahren zum Ermitteln eines Gehalts an Feststoffpartikeln in einem Flüssigkeitsstrom, vorzugsweise in einem Hydrauliksystem (2) mit folgenden Schritten:
- Reduzieren eines Gasgehalts an Gaspartikeln im Flüssigkeitsstrom,
- Komprimieren der Gaspartikel im Flüssigkeitsstrom.

2. Verfahren nach Anspruch 1, **gekennzeichnet durch** folgende Schritte:
- Ermitteln des Partikelgehalts an Feststoffpartikeln und Gaspartikeln im Flüssigkeitsstrom,
- Ermitteln des Gasgehalts im Flüssigkeitsstrom,
- Abschätzen der Plausibilität des ermittelten Partikelgehalts anhand des Gasgehalts, wobei der ermittelte Partikelgehalt als plausibel bewertet wird, wenn der Gasgehalt unterhalb eines vorbestimmten Gasgehaltgrenzwerts liegt.

3. Verfahren nach Anspruch 1, **gekennzeichnet durch** folgende Schritte:
- Identifikation störender Feststoffpartikel anhand der Partikelgröße, wobei ein störender Feststoffpartikel identifiziert wird, wenn die zugehörige Partikelgröße größer ist als eine vorbestimmte Partikelgrößengrenze, und wobei ein nicht störender Feststoffpartikel und/oder ein Gaspartikel identifiziert wird, wenn die zugehörige Partikelgröße kleiner ist als die Partikelgrößengrenze,
- Bestimmen des Gehalts der störenden Feststoffpartikel im Flüssigkeitsstrom.

4. Verfahren nach einem der Ansprüche 1 bis 3,
**dadurch gekennzeichnet,**
**dass** das Reduzieren des Gehalts an Gaspartikeln im Flüssigkeitsstrom mittels einer Entgasungseinrichtung erfolgt.

5. Verfahren nach Anspruch 4,
**dadurch gekennzeichnet,**
**dass** das Komprimieren der Gaspartikel im Flüssigkeitsstrom stromab der Entgasungseinrichtung erfolgt.

6. Verfahren nach einem der Ansprüche 1 bis 5,
**dadurch gekennzeichnet,**
**dass** das Komprimieren der Gaspartikel im Flüssigkeitsstrom durch eine Druckerhöhung im Flüssigkeitsstrom erfolgt.

7. Verfahren nach den Ansprüchen 5 und 6,
**dadurch gekennzeichnet,**
**dass** die Druckerhöhung mittels einer Pumpe erfolgt, die im Flüssigkeitsstrom stromab der Entgasungseinrichtung angeordnet ist.

8. Verfahren nach einem der Ansprüche 1 bis 7,
**dadurch gekennzeichnet,**
**dass** das Ermitteln des Partikelgehalts an Feststoffpartikeln und Gaspartikeln im Flüssigkeitsstrom mittels eines optischen Messverfahrens erfolgt, das mit Absorption und/oder Exstinktion arbeitet.

9. Verfahren nach einem der Ansprüche 1 bis 8,
**dadurch gekennzeichnet,**
**dass** das Ermitteln des Gasgehalts im Flüssigkeitsstrom mittels eines optischen Verfahrens erfolgt, das mit Streulicht arbeitet.

10. Vorrichtung zum Ermitteln eines Gehalts an Feststoffpartikeln in einem Flüssigkeitsstrom, vorzugsweise in einem Hydrauliksystem,
- mit einer Reduzierungseinrichtung (13) zum Reduzieren eines Gehalts an Gaspartikeln im Flüssigkeitsstrom,
- mit einer stromab der Reduzierungseinrichtung (13) angeordneten Kompressionseinrichtung (14) zum Komprimieren der Gaspartikel im Flüssigkeitsstrom,
- mit einer stromab der Kompressionseinrichtung angeordneten Messeinrichtung (15),
- mit einer Auswerteeinrichtung (16), die mit der Messeinrichtung (15) gekoppelt ist und so ausgestaltet und/oder programmiert ist, dass sie ein Verfahren nach einem der Ansprüche 1 bis 9 ausführen kann.

11. Vorrichtung nach Anspruch 10,
**dadurch gekennzeichnet,**
**dass** die Messeinrichtung (15) zum Ermitteln des Partikelgehalts an Feststoffpartikeln und Gaspartikeln im Flüssigkeitsstrom sowie zum Ermitteln des Gasgehalts im Flüssigkeitsstrom ausgestaltet ist.

12. Vorrichtung nach Anspruch 10,
**dadurch gekennzeichnet,**
**dass** die Messeinrichtung (15) zum Messen der Partikelgröße der Feststoffpartikel und der komprimierten Gaspartikel im Flüssigkeitsstrom ausgestaltet ist.

13. Maschine mit einem Hydrauliksystem (2) und mit einer Vorrichtung (8) nach einem der Ansprüche 10 bis 12.
